# EUROPEAN PATENT APPLICATION

(11) **EP 0 652 014 A1**
(43) Date of publication of application: **10.05.1995**
(21) Application number: 94308304.8
(22) Date of filing: 10.11.1994
(51) Int. Cl.: A61K 39/395, A61K 39/385, A61K 45/05

(54) **Treatment of prostatic hypertrophy**

(30) Priority: 10.11.1993 US 149986
(71) Applicant: NATIONAL INSTITUTE OF IMMUNOLOGY, New Delhi 110067 (IN)
(72) Inventor: Giri, Dipak Kumar, c/o National Institute, New Delhi 110067 (IN); Talwar, Gursaran Prasad, c/o National Institute, New Delhi 110067 (IN)
(74) Representative: Woods, Geoffrey Corlett

(57) **Abstract**

A method of for arresting the growth of both androgen-dependent and androgen-independent benign prostatic hypertrophy (BPH) and prostatic carcinoma in humans is disclosed using antibodies specifically reactive against human prostate specific antigen (PSA) is disclosed. The invention also includes the active immunization of male subjects against androgen-dependent and androgen-independent prostatic hypertrophy and hyperplasia by using a vaccine capable of inducing anti-PSA antibodies on administration, the vaccine containing PSA linked to an immunogenic carrier such as diphtheria toxin.

## Description

The present invention relates to the immunotherapy treatment of prostatic hypertrophy in males. More particularly, it relates to arresting or preventing the growth of androgen-dependent and/or androgen-independent benign prostatic hypertrophy or carcinoma cells in the (eg. human) prostate as well as to the immunization of males against both androgen-dependent and androgen-independent prostatic hypertrophy and hyperplasia.

The invention is based on the generation of antibodies specifically reactive against human prostate specific antigen (PSA) and their use in immunotherapy for stopping or preventing specifically the growth of prostate cancer cells. The invention also includes a vaccine consisting of a prostate specific antigen linked to an immunogenic carrier for the induction of antibodies reactive against prostate specific antigen (PSA).

The prostate, or more correctly prostate gland, is a pale, firm, partly muscular partly glandular body surrounding the base of the urinary bladder in man and other animals which discharges its viscid opalescent secretion through ducts opening into the floor of the urethra. With age, the prostate undergoes hypertrophy or enlargement leading to a fairly common and well-recognized syndrome known as benign prostatic hypertrophy (BPH). In fact, prostatic hypertrophy affects more than two thirds of all males over the age of fifty. Of those so affected, a percentage progresses to carcinoma of the prostate and indeed adenocarcinoma of the prostate is now the second highest killer of male human beings due to cancer.

Prostatic hypertrophy or hyperplasia as well as carcinoma of the prostate are to an extent believed to be dependent on the presence of the male sex hormone, testosterone. The existing therapy hitherto employed to treat prostatic hypertrophy is based on removal of the source of androgens. This therapy has included orchiectomy and/or treatment with anti-androgens and inhibitors of 5-α-reductase which convert testosterone into its active intracellular form dihydrotestosterone (DHT).

An alternative therapy recently introduced is to use an antagonist of the decapeptide LHRH, which by restricting androgen production, brings about therapeutic benefits. Immunization against LHRH by a vaccine (see UK Patent No. 2228262) also leads to marked atrophy of the prostate in rats and monkeys. The following references can be quoted in this connection:

N. Shastri, S.K. Manhar and G.P. Talwar, "Important role of the carrier in the induction of antibody response without Freund's complete adjuvant against a "self" peptide hormone LHRH", *Am. J. Reprod. Immunol*., **1**: 262-265 (1981).

G.P. Talwar, "Immunobiology of gonadotropin releasing hormone", *J. Steroid.* *Biochem.*, **23:** 795-800 (1985).

S.N. Upadhyay, A. Alam and G.P. Talwar, "Functional morphology of testis and its excurrent ducts in rats immunized with synthetic luteinizing hormone releasing hormone conjugated to tetanus toxoid", *J. Reprod. Immunol.*, **16**: 151-163 (1989).

R. Jayashankar, M.K. Chaudhuri, O. Singh, A. Alam and G.P. Talwar, "Semisynthetic anti-LHRH vaccine causing atrophy of the prostate", *Prostate,* **14** **(1)**: 3-11 (1989).

D.K. Giri, M.K. Chaudhuri, R. Jayashankar, G.S. Neelaram, S. Jayaraman and G.P. Talwar, "Histopathological changes in reproductive organs of male Wistar rats following active immunization against LHRH". *Expl. Mol. Pathol.* **52**: 54-62 (1990).

D.K. Giri, S. Jayaraman, G.S. Neelaram, R. Jayashankar and G.P. Talwar, "Prostatic hypoplasia in bonnet monkeys following active immunization with semisynthetic anti-LHRH vaccine". *Expl*. *Mol*. *Pathol*. **54:** 255-264 (1991).

E. Rovan, E. Fiebiger, N.R. Kalla, G.P. Talwar, W.Aulitzky, and J. Frick, "Effect of active immunization to luteinizing-hormone-releasing hormone on the fertility and histoarchitecture of the reproductive organs of male rat", *Urol Res*. **20:** 323-334 (1992).

However, in the course of clinical trials conducted in India and Austria with the vaccine of UK Patent No. 2228262, it was observed that immunization with this vaccine was beneficial to many but not all patients. Furthermore, the benefit was at best only temporary. The growth promoting role of androgens was curtailed but cancer cells were not killed or eliminated and the treatment had to be sustained.

Fortunately, as a result of the trials effected, it could be reasonably established that those that did not benefit had carcinoma which was androgen-independent since deprivation of androgens was of little avail. Given that carcinoma of the prostate is of both androgen-dependent and androgen-independent types, it becomes clear that any comprehensive treatment for arresting prostatic carcinoma and for immunization against prostatic hypertrophy and hyperplasia will necessitate a dual approach in order to suppress the growth of both types of prostatic cancer cells. At present, there is not specific drug or device available for the selective killing of prostatic carcinoma cells.

As its name suggests, prostate specific antigen (PSA) is a protein immunologically unique to the prostate. It is made and secreted exclusively by the epithelial cells lining the acini and the ducts of the prostate gland. It is present in seminal plasma. The serum level of PSA registers an increase in both benign and malignant growth of the prostate. The conclusion drawn from this is that there is a correlation between the synthesis and secretion of the protein and the prostatic cell mass. Accordingly, the quantum of PSA in serum is an indication of the extent of prostatic hypertrophy or hyperplasia and a fall in the level of the protein is considered an indication of a positive response to therapy.

PSA is already known and a method for purifying it is described in Wang *et al*, *Invest.Urol.* **17**:159-163 (1979).

One object of the present invention lies in the provision of a treatment for substantially arresting (or preventing) the growth of both androgen-dependent and androgen-independent prostatic hypertrophy and prostatic carcinoma in male human beings.

A further object of the invention is to immunize a male subject against prostate specific antigen, this being as a substitute for the presently utilized therapy or as a process to be employed in addition to the existing regimen.

Accordingly the present invention may generally provide a process for substantially arresting the growth of both androgen-dependent and androgen-independent benign prostatic hypertrophy and prostatic carcinoma in humans which comprises treating a male subject with antibodies specifically reactive against human prostate specific antigen.

The treatment can be of the passive kind employing polyvalent antibodies generated in any (suitable) mammal such as goats, sheep, rabbits or horses or employing monoclonal antibodies generated against PSA by hybrid cell clones or generated in bacteria by a genetic engineering route.

The invention also contemplates a process for the immunization of males against both androgen-dependent and androgen-independent prostatic hypertrophy and hyperplasia which comprises inducing in a male subject antibodies specifically reactive against human prostate specific antigen.

Since PSA is a "self" protein for humans in the same way as luteinizing hormone releasing hormone (LHRH) and human chorionic gonadotropin (hCG), the present invention has investigated a strategy similar to those employed in respect of LHRH and hCG to make PSA immunogenic in humans. This strategy involves conjugating the "self" protein to an immunogenic carrier. Examples of immunogenic carriers include tetanus toxoid, diphtheria toxoid and cholera toxin chain B as well as other similar immunogenic carriers. Specifically, the treatment comprises application of a vaccine active against prostate specific antigen linked to the immunogenic carrier, the vaccine inducing in the treated subject the formation of antibodies active against the antigen.

It has been established that such carrier conjugates can overcome immunological tolerance to the "self" protein in humans without entailing any side effects.

According to another modality, the antibody response to a "self" protein can be induced by expressing the gene for that protein in alignment with a transmembrane fragment in vaccinia, avian pox virus, adenovirus or salmonella as vectors (Patents applied for self hormone, hCG). Thus the present invention also envisages a live recombinant vaccine for immunizing males against both androgen-dependent and androgen-independent benign prostatic hypertrophy and hyperplasia which comprises vaccinia, avian pox virus, adenovirus or salmonella as vectors and in which the gene coding for prostate specific antigen is inserted in alignment with oligonucleotides coding for a transmembrane protein.

Both embodiments of the invention, namely passive administration of antibodies or active immunization can be viewed as substitutes for presently utilized therapy or as treatments to be employed in addition to the existing regimen.

Thus, in accordance with the first aspect of the present invention, there is provided the use of an antibody, or an immunoreactive fragment of mutant thereof, specific for prostate specific antigen (PSA) in the preparation of a medicament for the prophylaxis or treatment of both androgen-dependent and androgen-independent benign prostatic hypertrophy (BPH), prostatic carcinoma and/or prostatic hyperplasia.

The antibody may be a polyclonal antibody, such as from a goat, sheep, rabbit or horse, the first of these being preferred. Alternatively one can employ an monoclonal antibody or fragment thereof, for example generated by a hybrid cell clone.

A second aspect of the invention relates to a pharmaceutical composition, preferably adapted for parenteral administration, comprising an antibody, or an immunoreactive fragment or mutant thereof, specific for PSA as defined in the first aspect and pharmaceutically acceptable carrier, where the composition is adapted to give a dose of from 25 to 500µg. Preferably the composition is adapted to give a dose of from 150 to 125µg and/or 100µg twice weekly.

A third aspect of the invention relates to PSA, or an immunogenic fragment or mutant thereof, linked to an immunogenic carrier. The carrier can be a tetanus toxoid, diphtheria toxoid or cholera toxin chain B. Diphtheria toxoid is preferred.

By "fragment" one means a portion of the original molecule (such as the antibody or PSA, as appropriate) provided that the fragment retains all or substantially all of the activity of relevance ascribed to it (such as the ability to be specific for, or react with, PSA in the case of antibodies, or to be immunogenic, that is to say, it can induce antibodies *in vivo*).

Mutants involve the addition, deletion, and/or substitution of one or more amino acid residues provided that the activity of the molecule is not, or not substantially, altered. In the case of antibodies, the mutant should still be specific for that antigen (such as PSA). In the case of mutant PSA, such mutants preferably still be immunogenic.

A fourth aspect of the present invention relates to pharmaceutical compositions, for example vaccines, that comprise the PSA (or it's immunogenic fragment or mutuant) when linked to an immunogenic carrier, and a pharmaceutically acceptable excipient.

A fifth aspect of the present invention relates to PSA, or immunogenic fragment or mutant thereof, when linked to an immunogenic carrier, for use in a method of prophylaxis of treatment of the human or animal body by therapy.

In addition, a sixth aspect of the invention relates to the use of PSA, or an immunogenic fragment or mutant thereof, linked to an immunogenic carrier for use in the preparation of a medicament for the prophylaxis or treatment of both androgen-dependent and androgen-independent benign prostatic hypertrophy, prostatic carcinoma and/or prostatic hyperplasia.

A further aspect of the present invention relates to a product comprising PSA, or immunogenic fragment of mutant thereof, which is linked to a immunogenic carrier and either an anti-androgen or an inhibitor of 5-α-reductase as a combined preparation for separate, simultaneous or sequential use in the prophylaxis or therapy of both androgen-dependent and androgen-independent BPH, prostatic carcinoma and/or prostatic hyperplasia.

The invention will now be described in detail in the following non-limiting Examples which refer to the Figures in the accompanying drawings in which:
Figure 1 constitutes four comparative photo-micrographs of culture plates showing the effect of antibodies reactive against PSA at different doses on the proliferation of androgen-dependent LNCaP prostatic carcinoma cells;
Figure 2 is a bar graph of % inhibition against anti-PSA antibody dose showing the effect of the antibodies on the proliferation of androgen-dependent LNCaP prostatic carcinoma cells in a dose-dependent manner;
Figure 3 is a bar graph of absorbance (formazan production) against anti-PSA antibody dose showing the effect of the antibodies on the proliferation of androgen-dependent LNCaP prostatic carcinoma cells in a dose-dependent manner;
Figure 4 is a bar graph of the absorbance against PSA concentration showing the effect of pretreatment of anti-PSA antibodies (4µg) with PSA on the proliferation of androgen-dependent LNCaP prostatic carcinoma cells. Control (C) wells received no anti-PSA antibodies;
Figure 5 is a bar graph of absorbance against anti-PSA antibody dose showing the effect of the antibodies on the proliferation of laryngeal carcinoma cells (HEp-2) at various doses;
Figure 6 constitutes four comparative photo-micrographs of culture places showing the effect of antibodies reactive against prostate specific antigen (PSA) at different doses on the proliferation of androgen-independent DU-145 prostatic carcinoma cells;
Figure 7 is a bar graph of % inhibition against anti-PSA antibody dose showing the effect of anti-PSA antibodies on the proliferation of androgen-independent DU-145 prostatic carcinoma cells in a dose-dependent manner;
Figure 8 constitutes four photographs of an athymic nude mouse implanted subcutaneously with androgen-independent DU-145 human carcinoma cells, the photographs showing the growth of the resulting tumour at 6, 8, 12 and 14 weeks respectively;
Figure 9 constitutes 12 photographs of four athymic nude mice at 6, 8, 12 and 14 weeks showing the inhibitory effect on the growth of DU-145 cells incubated with varying doses of anti-PSA antibodies at 37°C for one hour prior to subcutaneous implantation. Each mouse subsequently received passively that same indicated dose of antibodies twice weekly for a period of 10 weeks;
Figure 10 constitutes 12 photographs of four athymic nude mice bearing an induced androgen-independent DU-145 cell tumour which were subsequently injected with varying indicated doses of anti-PSA antibodies twice weekly to bring about necrosis of tumour over a eight week treatment period;
Figures 11A and 11B are histological photographs of a carcinoma tumour (A) treated for eight weeks with anti-PSA antibodies and of a similar control tumour (B) also treated for eight weeks, with saline;
Figure 12 is a graph of OD at 490nm (i.e. anti-PSA antibody titres) against time of four male bonnet monkeys immunised with a vaccine according to the invention at various times (indicated by the arrows);
Figure 13 constitutes two graphs of testosterone levels against time for control monkeys and monkeys immunised with a vaccine according to the invention;
Figure 14 is a bar graph of the group mean percentage weights of reproductive organs (key given in Example 5) from control monkeys and monkeys immunised with a vaccine according to the invention; and
Figure 15 constitutes four histological photomicrographs of prostates from, respectively, a control monkey and three monkeys immunised with a vaccine according to the invention.

### EXAMPLE 1

### In VitroTreatment of Androgen-Dependent Human Prostate Carcinoma Cells with Anti-PSA Antibodies

### Method A

Human prostate carcinoma cells derived from metastatic foci in lymph nodes [LNCaP] were cultured *in vitro* in RPMI 1640 medium (GIBCO) supplemented with 7% heat inactivated (30 mins., 56°C) fetal calf serum (GIBCO), penicillin (100 units/ml) and streptomycin (100 µg/ml) at 37°C in a humidified atmosphere of 5% CO₂ in air. These carcinoma cells, which constitute an androgen-dependent cell line, were obtained from the American Type Culture Collection [ATCC], U.S.A. from deposit no. ATCC CRL-1740. The cells are spindle-shaped and the doubling time is about 36 hours. Two separate sets of culture places were set up.

In a separate operational procedure, polyvalent antibodies reactive against prostate specific antigen (PSA) were raised in goats. The prostate specific antigen was purified from human seminal plasma by the two step purification procedure using an ion-exchange column and a gel filtration column. Chromatographic separation was carried out using a DEAE-Sepharose™ fast flow column followed by Sephacryl™ S-100 column. The purity of PSA was verified by sodium dodecyl sulphatepolyacrylamide gel electrophoresis (SDS-PAGE). A single 34kD protein band was observed by Coomassie and silver stains. Immunization was carried out in goats with purified PSA (100 µg/ml), emulsified with an equal volume of complete Freund's adjuvant (CFA) intradermally, followed by two monthly injections of 50 µg PSA in incomplete Freund's adjuvant (IFA). Booster injection of 25 µg PSA in IFA was given after 8 weeks. Immunoglobulins were precipitated at 40% ammonium sulphate saturation and resuspended in phosphate buffered saline (PBS). The antigen-binding capacity was determined by ELISA.

To the first of the experimental culture plates the antibodies generated in the separate operational procedure described, having antigen-binding capacity of 1100 µg/ml, were added at concentrations of 0.5 µg, 1 µg, 2 µg, 4 µg, 8 µg and 16 µg per culture well. Likewise, to the second set of control culture plates there were added equal volumes of pre-immune goat serum which did not contain these antibodies.

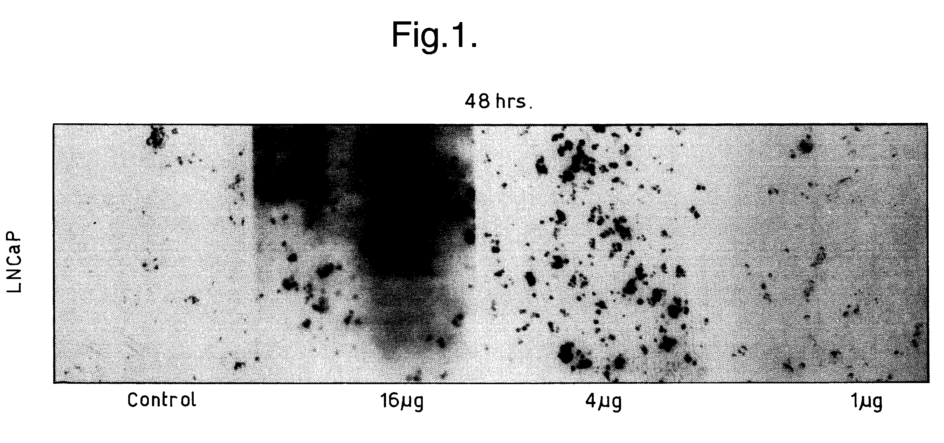

At the end of 24 hours, partial growth inhibitory effects of the antibodies were observed in respect of the experimental culture plates and by 48 to 72 hours almost all, i.e. 100%, of the cells were lysed. The comparative situation with respect to the control culture plates can be observed from Figure 1 which is four photo-micrographs taken after 48 hours of culture. The control culture plates are shown at the left of the Figure while the position of three separate wells of the experimental culture plates to which 1 µg, 4 µg and 16 µg antibodies respectively were added can be observed in the middle and on the right of the Figure. The photo-micrographs establish that the effect of the antibodies on the carcinoma cells is dose dependent.

### Method B

Confirmation of the nocive effect of these anti-PSA antibodies on the growth of human prostate cancer cells was further confirmed by two criteria. It is recognized that the DNA of a cell has to double before the cell divides. Thymidine is one of the building blocks of DNA and growth cells incorporate it actively. According to the first criterion, ³[H] thymidine uptake was measured after 24, 48 and 72 hours of exposure to the antibodies using a standard ³[H] thymidine incorporation assay. The inhibition of the synthesis of DNA was evident from the decline of ³[H] thymidine incorporation of adequate concentration of antibodies in LNCaP cells. The CPM for LNCaP declined to background counts at 8 and 16 µg immunoglobulins. This effect was caused in absence of exogenous complement but was augmented in presence of complement. The growth inhibitory effect was not due to any non-specific components of the goat serum as preimmune serum manifested no action. This effect was related dose-wise to anti-PSA antibodies.

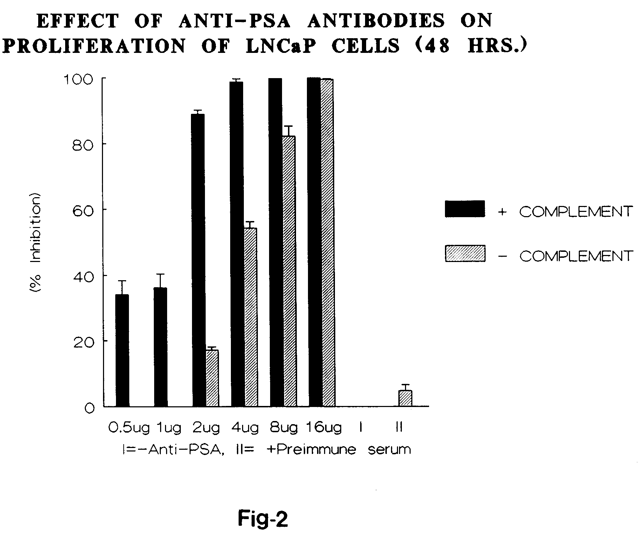

On the other hand, antibodies raised in goats against a non-related antigen such as nuclear polyhedrosis baculovirus (NPB) had no effect. Furthermore, in respect of the control culture plates of carcinoma cells to which no antibodies active against PSA were added, the presence of thymidine was clearly observable. In contradistinction, the experimental culture plates in which antibodies active against PSA were added failed to reflect the incorporation of ³[H] thymidine thus providing evidence for the lack of DNA synthesis in the treated cells. As stated above, the effect of the antibodies was dose dependent. Complete inhibition and death of carcinoma cells were attained with a dose of 4 µg and above of antibody concentration, whereas partial action proportional to the dose of antibodies was observed at concentrations lower than 4 µg. These effects of anti-PSA antibodies at various doses is shown in Figure 2. The complement employed with some of the antibody doses was freshly prepared from rabbit serum, and enhances antibody binding. The pre-immune serum was taken from goats prior to immunisation (to prepare the polycolonal antibodies).

### Method C

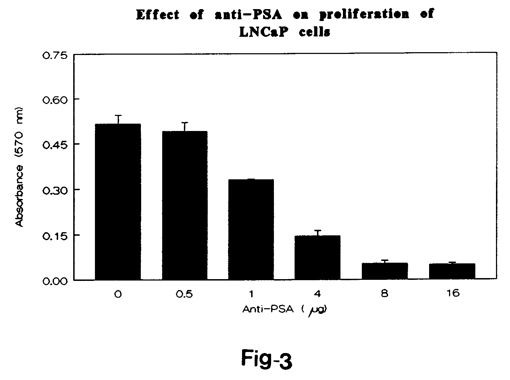

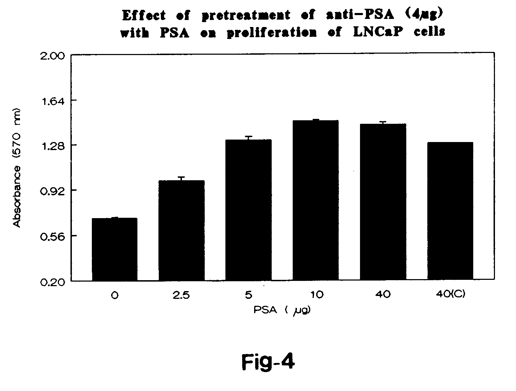

According to the second criterion employed, the anti-proliferative effect of the antibodies was measured by a non-radioactive cell proliferation assay kit (Promega, Madison, USA). This assay, originally described by Mosmann, (T. Mosmann, *J*. *Immunol Meth*.**65**, 55, 1983) is based on the principle that the living cells convert the tetrazolium salt of dye solution into a formazan product that upon solubilization has colour and is quantifiable by absorbance. LNCaP cells were exposed to increasing concentrations of anti-PSA antibodies for 24 hrs. The amount of coloured product, which reflects the population of living cells as estimated by ELISA reader, was remarkably reduced at 8 and 16 µg dose levels of antibodies (Figure 3). The cytotoxic effect of antibodies (4 µg) was absorbable (or neutralised) by varying concentrations of PSA (Figure 4) implying thereby that antibodies directed against PSA were exercising nocive action.

LNCaP cells treated with anti-PSA antibodies for 24 hours or more and then replaced with fresh medium failed to grow. Microscopically, cytoplasmic granulation and vacuolation, condensation of nuclear chromatin, and disintegration of plasma membrane (that finally leads to fragmentation) were observable.

### Method D

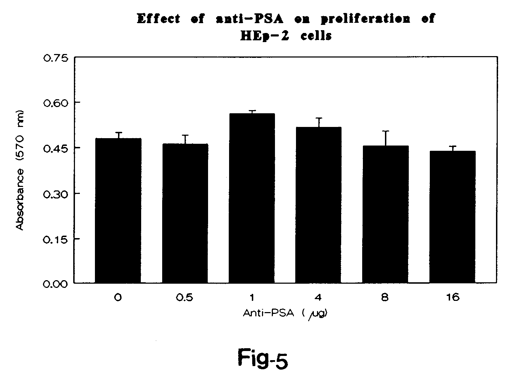

Parallel experiments employing the laryngeal carcinoma cell line (HEp-2) clearly demonstrated that anti-PSA antibodies had no effect on the viability and multiplication of these cells (Figure 5). This observation indicates that anti-PSA antibodies acted on LNCaP cells, (which secrete PSA), but not on non-PSA secretory tumour cells such as HEp-2.

### EXAMPLE 2

### In VitroTreatment of Androgen-Independent Human Prostate Carcinoma Cells with Anti-PSA Antibodies

### Method A

DU-145 human prostate carcinoma cells derived from metastatic foci in the brain of a human prostate carcinoma patient were cultured *in vitro* in Dulbecco's modified Eagle's medium (GIBCO) supplemented with 10% heat inactivated fetal calf serum, penicillin and streptomycin. The carcinoma cells which constituted an androgen-independent cell line were obtained from the American Type Culture Collection (ATCC), U.S.A. from deposit number ATCC HTB-81.

As in the case of Example 1, Method A, two separate sets of culture plates were set up. The first experimental culture plates were treated with the antibodies generated in goats described earlier (specifically reactive against PSA) at concentrations of 0.5 µg, 1 µg, 2 µg, 4g, 8µg and 16µg per culture well. The second set of control culture plates were treated with equal volume of pre-immune goat serum which did not contain such antibodies.

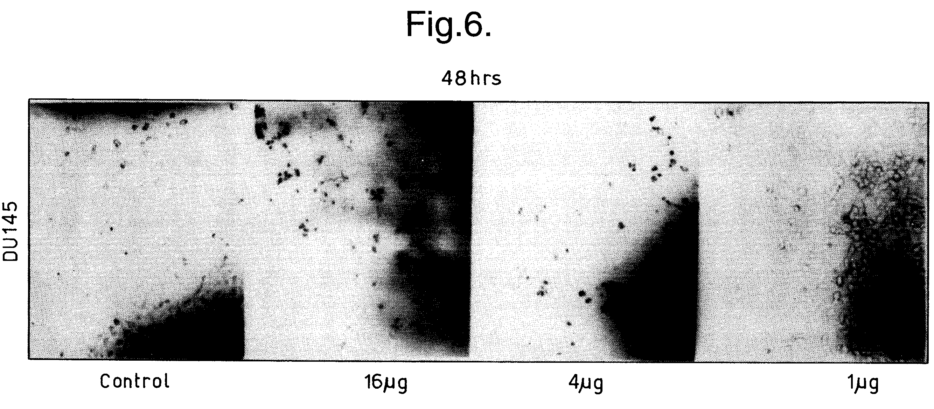

The comparative situation between the control and the experimental culture plates after 48 hours of culture can be observed from the photomicrographs in Figure 6. The photograph to the left of the Figure is representative of the control culture plates and considerable cell growth can be observed therefrom. The position regarding the separate wells of the experimental culture plates to which 1 µg, 4µg and 16 µg antibodies respectively have been added can also be observed in the Figure. The photomicrographs establish that the effect of the antibodies on the carcinoma cells is dose dependent.

### Methods B and C

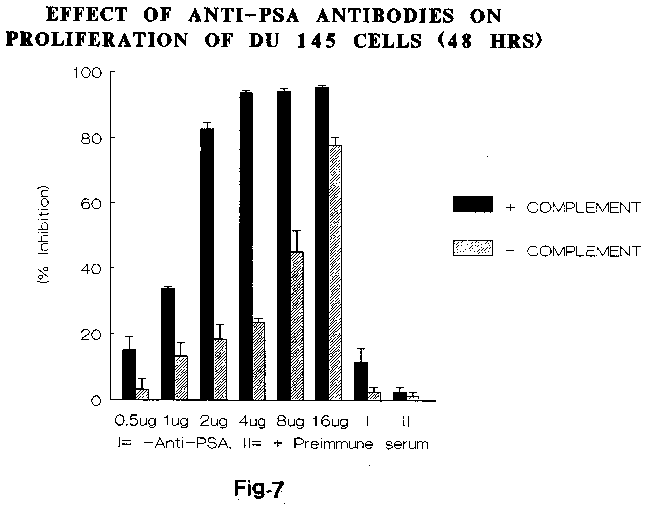

The same two confirmatory criteria described in Example 1 were followed in this Example as well. The control culture plates of DU-145 carcinoma cells to which antibodies active against PSA were not added revealed the incorporation of thymidine establishing the active involvement of DNA synthesis. On the other hand, the experimental culture plates to which the antibodies active against PSA were added failed to reflect the incorporation of thymidine thus providing evidence of lack of DNA in treated cells. As before, the effect of the antibodies was dose dependent. Complete inhibition and death of carcinoma cells was attained with a dose of 4 µg and above of antibody whereas partial action proportional to the dose was observed at concentrations lower than 4µg. These effects of anti-PSA antibodies at various doses are shown in Figure 7. The legend for this Figure is the same as for Figure 2.

### EXAMPLE 3

### In Vivo(and pre-) Treatment of Prostatic Cancer Cells with Anti-PSA Antibodies

Athymic nude mice [NIH (Swiss) nu/nu], which are deprived of thymus function and do not reject implants of human tumour cells were isolated into 4 groups for evaluation of the effect of anti-PSA antibodies at 4 doses.

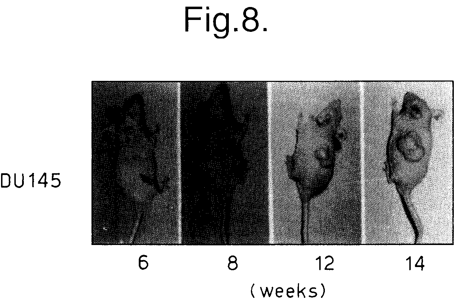

In a first series of investigations, androgen independent carcinoma cells (DU-145) were implanted (1.08 x 10⁷ cells) subcutaneously under the dorsal skin. The tumour cells grew into a tumour mass over time as observable in the photographs constituting Figure 8.

In a parallel run, the same number of carcinoma cells were incubated for 1 hour at 370C with increasing amounts of anti-PSA antibodies ranging from 1 to 125 µg and then implanted in groups of three nude mice at each concentration. The mice were further administered the same indicated dose of antibodies near the site of tumour growth twice weekly.

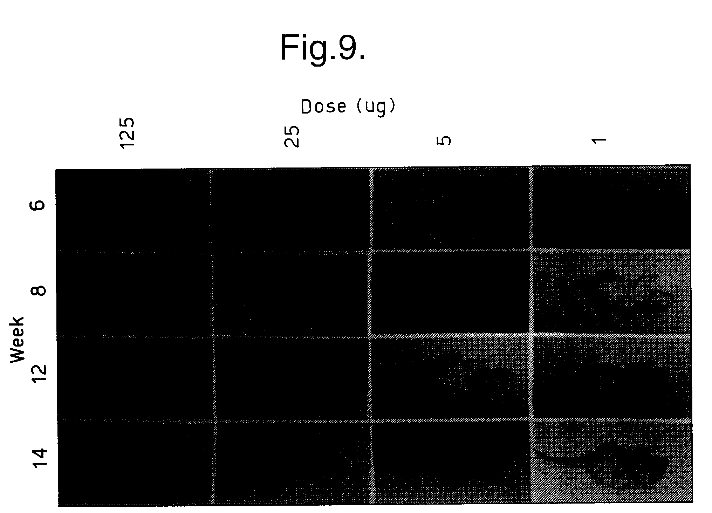

The (16) photographs constituting Figure 9 show the status of the tumour growth in these mice at 6, 8, 12, and 14 weeks for antibody doses of 1 µg, 5 µg, 25 µg and 125 µg respectively. It is clearly evident that anti-PSA antibodies completely stopped the growth of the tumour at adequate concentrations and that the effect was dose dependent.

### EXAMPLE 4

### In VivoTreatment of Prostatic Cancer Cells with Anti-PSA Antibodies

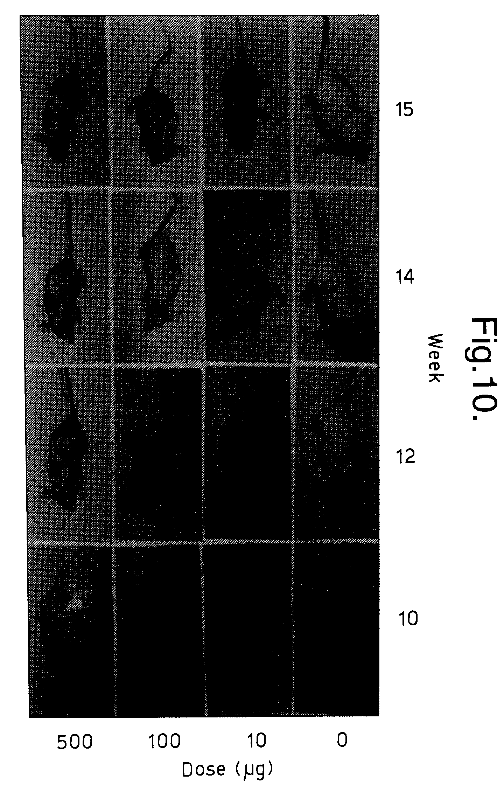

In another series of experiments, the anti-PSA antibodies were administered passively after the tumour had grown to a mass. The treatment caused necrosis of the tumour characterized by discolouration of the skin with development of cavities that had widened in the course of time (Figure 10).

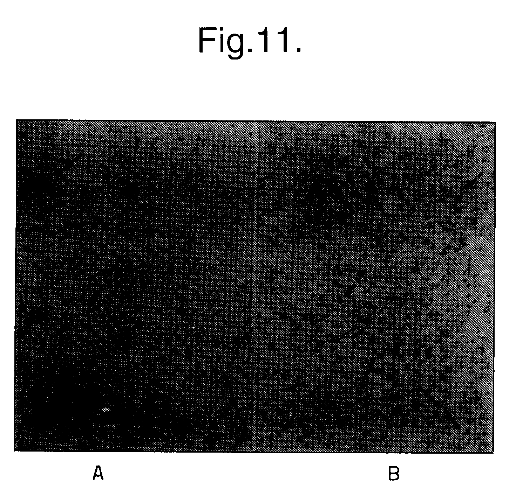

Histological examination (Figure 11) showed that the tumour cells were completely eliminated from the tumour mass (indicated by arrow heads) while the supporting stromal cells remained undamaged (Figure 11A). Figure 11B gives the comparative histology of a tumour in a control animal given only phosphate buffered saline (PBS). The tumour cells are in an active stage of multiplication as evident from the mitotic indices (arrows).

These experiments confirm the anti-prostatic cancer cell activity of the antibodies reacting with PSA *in vivo.*

### EXAMPLE 5

### In VivoInduction of Anti-bodies Against PSA In Monkeys Using A Vaccine

Prostate specific antigen (PSA) was conjugated to diphtheria toxoid using methods previously described (Singh *et al*, *Fertil Steril* **53**: 739-744) (1989), Gaur et al Int. *Immunol*, **2**: 151-155 (1990) and Talwar *et al Proc*. *Natl Acad Sci USA* **91**: 8532-8536 (1994). The conjugate was used to prepare a vaccine that was employed to immunise bonnet monkeys. The monkey prostate antigen cross reacts with antibodies against the human PSA. Hence, these animals could be utilised to determine the effect of immunisation with such vaccines on the prostate size of the animal. The studies conducted demonstrated the lack of effects of immunisation with such vaccines on the health and various metabolic and endocrine functions of the animal. No significant difference in the weights of the monkeys occurred as a result of immunisation during the observation period.

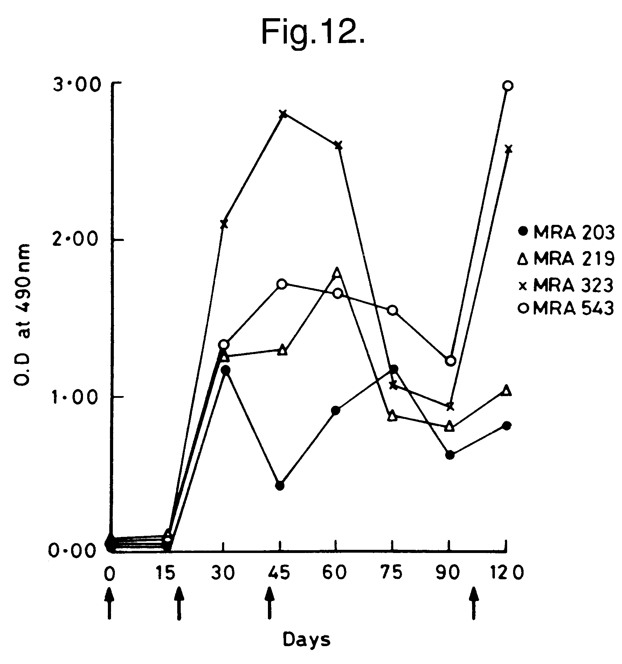

Figure 12 is a graph of optical density against time following the first immunisation. The optical density is an indication of the anti-PSA antibody titres from the serum of four male bonnet monkeys. Each monkey is identified by the legend MRA (short for *Macaca radiata* monkey species) followed by a three digit number identifying the particular monkey. Each monkey was immunised with the PSA-DT vaccine preparation on day 0 (100µg dose), day 18 (50µg), day 43 (25µg) and on day 101 or 102 (25µg), these points in time being indicated on the graph of Figure 12 by arrows. The antibody titres are presented as 1:10,000 dilution of sera samples. Thus Figure 12 gives the antibody kinetics in four monkeys immunised with the vaccine of the invention. It will be seen that the vaccine was able to induce anti-PSA antibodies *in vivo*.

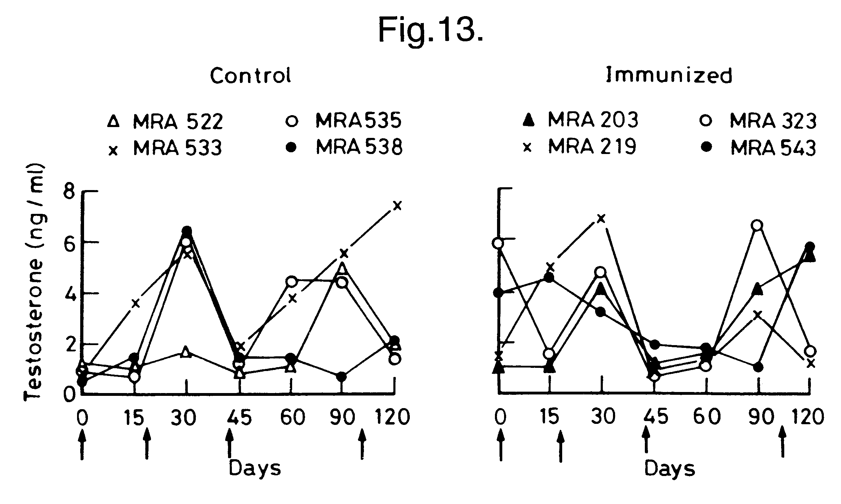

Simultaneously four additional male monkeys were observed as controls, and were only immunised with the carrier, diphtheria toxoid. Antibody titre results for the controls are not presented, although testosterone levels of the four control monkeys and the four previously immunised monkeys (numbers 213, 219, 323 and 543) were measured. Testosterone serum levels were determined at day 0 and at every 15 days thereafter up to 120 days, the results of which are presented in Figure 13. The arrows again indicate days on which the vaccine (or diphtheria toxoid alone was administered and are the same as shown in Figure 12). Both the vaccine or carrier only were administered by injection.

Fluctuations in the blood testosterone level are normally encountered in these animals and the levels were found to be within the normal range in both the control as well as the immunised monkeys. This demonstrates that the vaccine had little if any impact, and did not influence testosterone levels.

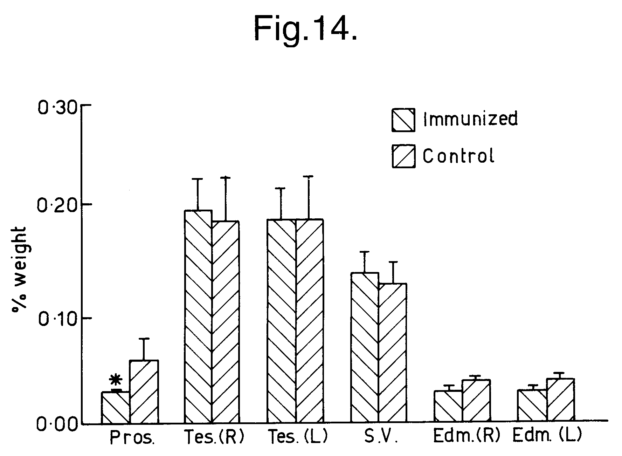

At the end of four months both the control and immunised monkeys were euthanized (sacrificed). The reproductive organs were weighed and their percentage in relation to their entire body weight of the monkey calculated. The results are presented in Figure 14 which shows the group mean percent weights of the reproductive organs from both PSA-DT immunised monkeys, and control monkeys. The key to the abbreviations in Figure 14 are as follows:
Pros - Prostate;
Tes.(R) - right testis;
Tes.(L) - left testis;
S.V. - seminal vesicles;
Edm.(R) - right epididymis;
Edm. (L) - left epididymis.

No significant difference was noted in the percentage weight of the testes, epididymis and seminal vesicles, accessory reproductive organs of monkeys. However, note that the weights of the prostates from immunised monkeys were significantly reduced (see the asterisk in Figure 14) in comparison to that in control monkeys.

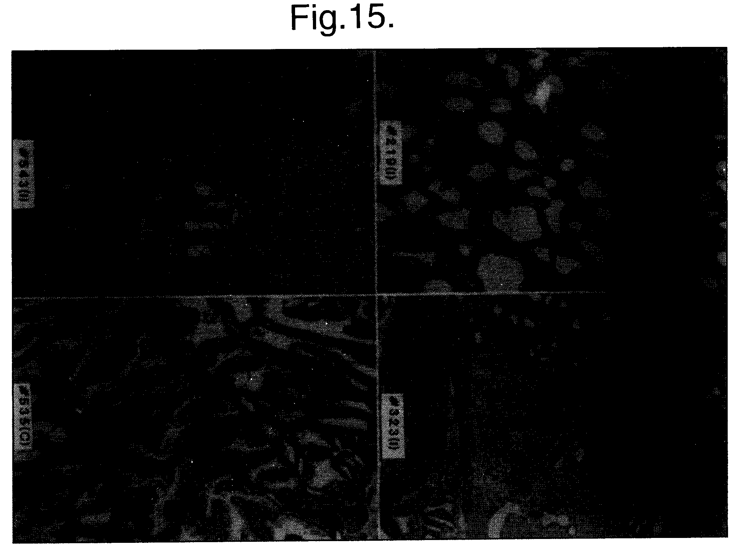

The autopsies performed did not reveal any gross pathology of the monkeys immunised with the PSA vaccine. Figure 15 gives four photomicrographs for histological purposes of prostates from one of the control monkeys (MRA 535, additionally labelled (C) in Figure 15), as well as from three of the monkeys that were immunised with the vaccine. The histomorphology of prostates are shown using the staining method H&E (haematoxylin and eofin) x 10. From the photomicrographs the histomorphology of the prostates from immunised monkeys showed hyperplasia characterised by reduction of acinar size and absence of branching patterns of glandular units associated with flattening of lining epithelium, as compared to the prostates from control monkeys.

This Example demonstrates the efficacy of the vaccine in diminishing the size of the prostatic tissue without ill effects on other organs and body functions.

### EXAMPLE 6

### Preparation of Anti-PSA Monoclonal Antibodies

Monoclonal antibodies are prepared using the classic method originally expounded by Köhler & Milstein. A suitable mammal, such as a mouse, is immunised with prostate specific antigen (PSA) prepared as described in Example 1, Method A. Its spleen is removed several weeks later. *In vitro* a mixture of lymphocytes and spleen plasma cells is fused with myeloma cells by exposing them to polyethylene glycol (which induces cell fusion). A mutant myeloma cell line lacking HGPRT can be used to enable selection of hybrid cells from a HAT medium. Hybrid cells are screened to determine those that produce anti-PSA antibodies. Supernatant from wells in which the hybridoma cells are cultured are screened for the presence of anti-PSA antibodies and the cells in positive wells are cloned and screened again to obtain hybridomas of a single type.

## Claims

1. The use of an antibody, or an immunoreactive fragment of mutant thereof, specific for prostate specific antigen (PSA) in the preparation of a medicament for the prophylaxis or treatment of both androgen-dependent and androgen-independent benign prostatic hypertrophy (BPH), prostatic carcinoma and/or prostatic hyperplasia.

2. The use according to claim 1 wherein the antibody is a polyclonal antibody, preferably from a goat, sheep, rabbit or horse.

3. The use according to claim 1 wherein the antibody or fragment thereof is, or is a fragment of, a monoclonal antibody.

4. A pharmaceutical composition, preferably adapted for parenteral administration, comprising an antibody, or an immunoreactive fragment or mutant thereof, specific for PSA as defined in any of claims 1 to 4 and present at from 25 to 500µg and a pharmaceutically acceptable carrier.

5. PSA, or an immunogenic fragment or mutant thereof, linked to an immunogenic carrier.

6. A pharmaceutical composition, such as a vaccine, comprising PSA, or an immunogenic fragment or mutant thereof, linked to an immunogenic carrier, and a pharmaceutically acceptable excipient.

7. A composition according to claim 6 wherein the immunogenic carrier is a tetanus toxoid, diphtheria toxoid or cholera toxin chain B.

8. PSA, or an immunogenic fragment or mutant thereof, linked to an immunogenic carrier for use in a method of prophylaxis or treatment of the human or animal body by therapy.

9. The use of PSA, or an immunogenic fragment or mutant thereof, linked to an immunogenic carrier for use in the preparation of a medicament for the prophylaxis or treatment of both androgen-dependent and androgen-independent benign prostatic hypertrophy, prostatic carcinoma and/or prostatic hyperplasia.

10. A product comprising PSA, or immunogenic fragment of mutant thereof, which is linked to a immunogenic carrier and either an anti-androgen or an inhibitor of 5-α-reductase as a combined preparation for separate, simultaneous or sequential use in the prophylaxis or therapy of both androgen-dependent and androgen-independent BPH, prostatic carcinoma and/or prostatic hyperplasia.
